# EUROPÄISCHE PATENTANMELDUNG

(11) **EP 2 594 252 A2**
(43) Veröffentlichungstag der Anmeldung: **22.05.2013**
(21) Anmeldenummer: 12180503.0
(22) Anmeldetag: 15.08.2012
(51) Int. Cl.: A61K 8/41, A61Q 5/00

(54) **Haarbehandlungsmittel mit kationischem Pflegestoff und Tensid(en)**

(30) Priorität: 23.09.2011 DE 102011083313
(71) Anmelder: Henkel AG & Co. KGaA, 40589 Düsseldorf (DE)
(72) Erfinder: Krueger, Marcus, 25373 Ellerhoop (DE)

(57) **Zusammenfassung**

"Haarbehandlungsmittel mit kationischem Pflegestoff und Tensid(en)" Haarbehandlungsmittel, die verbesserte Pflegeeffekte, insbesondere verbesserte Kämmbarkeiten, Weichheit und Glanz des Haares, mit einer Viskositätsstabilität über weite Temperaturbereiche verbinden, enthalten - bezogen auf ihr Gewicht - 0,1 bis 40 Gew.-% anionische(s) und/oder amphotere(s) und/oder nichtionische(s) Tensid(e) sowie 0,1 bis 30 Gew.-% mindestens einer Verbindung der allgemeinen Formel (I) 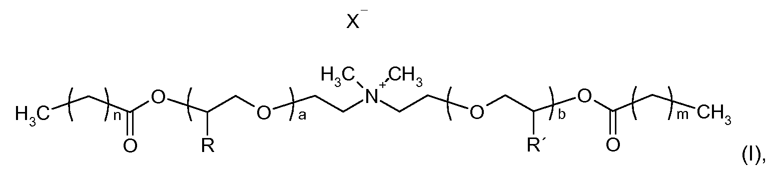 in der
n und m
unabhängig voneinander für ganze Zahlen zwischen 5 und 40 stehen, mit der Maßgabe, daß n + m ≥ 38 ist;
a und b
unabhängig voneinander für ganze Zahlen zwischen 1 und 10 stehen;
R und R'
unabhängig voneinander ausgewählt sind aus -H und -CH₃;
X-
ein physiologisch verträgliches Anion ist.

## Beschreibung

Die Erfindung betrifft Haarbehandlungsmittel, welche kationische(n) Pflegestoff(e) und bestimmte Tensid(e) enthalten sowie die Verfahren zur Reinigung und/oder Pflege von Haar unter Einsatz dieser Mittel.

Die kosmetische Behandlung von Haut und Haaren ist ein wichtiger Bestandteil der menschlichen Körperpflege. So wird menschliches Haar heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Nicht zuletzt durch die starke Beanspruchung der Haare, beispielsweise durch das Färben oder Dauerwellen als auch durch die Reinigung der Haare mit Shampoos und durch Umweltbelastungen, nimmt die Bedeutung von Pflegeprodukten mit möglichst langanhaltender Wirkung zu. Derartige Pflegemittel beeinflussen die natürliche Struktur und die Eigenschaften der Haare. So können anschließend an solche Behandlungen beispielsweise die Naß- und Trockenkämmbarkeit des Haares, der Halt und die Fülle des Haares optimiert sein oder die Haare vor erhöhtem Spliß geschützt sein. Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern. Diese Präparate enthalten neben den üblichen Komponenten, beispielsweise zur Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate wirken im allgemeinen bevorzugt an der Haaroberfläche. So sind Wirkstoffe bekannt, welche dem Haar Glanz, Halt, Fülle, bessere Naß- oder Trockenkämmbarkeiten verleihen oder dem Spliß vorbeugen. Genauso bedeutend wie das äußere Erscheinungsbild der Haare ist jedoch der innere strukturelle Zusammenhalt der Haarfasern, der insbesondere bei oxidativen und reduktiven Prozessen wie Färbung und Dauerwellen stark beeinflußt werden kann.

Die bekannten Wirkstoffe können jedoch nicht alle Bedürfnisse in ausreichendem Maße abdecken. Es besteht daher weiterhin ein Bedarf nach Wirkstoffen bzw. Wirkstoffkombinationen für kosmetische Mittel mit guten pflegenden Eigenschaften und guter biologischer Abbaubarkeit. Insbesondere in farbstoff- und/oder elektrolythaltigen Formulierungen besteht Bedarf an zusätzlichen pflegenden Wirkstoffen, die sich problemlos in bekannte Formulierungen einarbeiten lassen.

Wegen der Affinität zur Keratinfaser haben kationische Pflegestoffe eine besondere Bedeutung. Zahlreiche Stoffe aus den verschiedensten Gruppen sind bekannt und werden breit in kosmetischen Mitteln eingesetzt.

So offenbart die EP 951 898 B1 haarkonditionierende Mittel, welche quaternäre Ammoniumverbindung aus der Gruppe der Esterquats und mindestens eine Silikonverbindung enthalten.

Diesterquats, d.h. quaternäre Ammoniumverbindungen mit zwei Acylresten im Molekül, werden beispielsweise in der EP 918 743 offenbart.

Die WO 2004/093834 A1 offenbart spezielle Diesterquats, die auf Diolen anstelle von Ethanolaminen basieren und langkettige Acylreste aufweisen, welche ggf. über PEG- bzw. PPG-Gruppierungen an die Diolgruppierungen gebunden sein können.

Es bestand daher die Aufgabe, Haarbehandlungsmittel bereitzustellen, die verbesserte Pflegeeffekte, insbesondere verbesserte Kämmbarkeiten, Weichheit und Glanz des Haares, mit einer Viskositätsstabilität über weite Temperaturbereiche verbinden.

Es wurde nun gefunden, daß die Kombination von kationischen Pflegestoffen und bestimmten Tensiden die genannten Vorteile realisieren kann.

Gegenstand der vorliegenden Erfindung ist in einer ersten Ausführungsform ein Haarbehandlungsmittel, enthaltend - bezogen auf sein Gewicht -
a) 0,1 bis 40 Gew.-% anionische(s) und/oder amphotere(s) und/oder nichtionische(s) Tensid(e),
b) 0,1 bis 30 Gew.-% mindestens einer Verbindung der allgemeinen Formel (I) 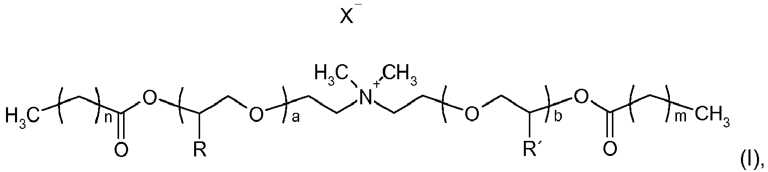
in der
- n und m: unabhängig voneinander für ganze Zahlen zwischen 5 und 40 stehen, mit der Maßgabe, daß n + m ≥ 38 ist;
- a und b: unabhängig voneinander für ganze Zahlen zwischen 1 und 10 stehen;
- R und R': unabhängig voneinander ausgewählt sind aus -H und -CH₃;
- X-: ein physiologisch verträgliches Anion ist.

Haarbehandlungsmittel im Sinne der vorliegenden Erfindung sind beispielsweise Haarfärbemittel, Blondiermittel, Haarshampoos, Haarkonditionierer, konditionierende Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen. Bevorzugte erfindungsgemäße Mittel sind Shampoos, Konditioniermittel oder Haar-Tonics.

Als ersten wesentlichen Inhaltsstoff enthalten die erfindungsgemäßen Mittel 0,1 bis 40 Gew.-% anionische(s) und/oder amphotere(s) und/oder nichtionische(s) Tensid(e). Vorzugsweise werden das bzw. die Tensid(e) innerhalb engerer Mengenbereiche eingesetzt, so daß bevorzugte erfindungsgemäße Haarbehandlungsmittel dadurch gekennzeichnet sind, daß sie 2,5 bis 35 Gew.-%, vorzugsweise 3 bis 30 Gew.-%, weiter bevorzugt 5 bis 29 Gew.-%, noch weiter bevorzugt 7,5 bis 28 Gew.-% und insbesondere 12,5 bis 27 Gew.-% anionische(s) und/oder amphotere(s) und/oder nichtionische(s) Tensid(e) enthalten.

Je nach Anwendungszweck kann dabei der Gehalt an Tensiden aus den einzelnen Gruppen variieren, wobei anionische(s) Tensid(e) in reinigenden Formulierungen (insbesondere in Shampoos) besonders bevorzugt sind.

Als anionische Tenside und Emulgatoren eignen sich für die erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside und Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Acylglutamate der Formel (I), 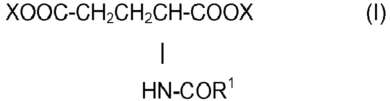
in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (II), 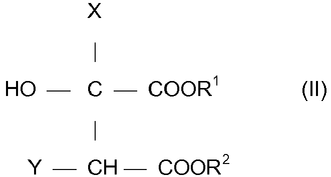 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist,
- Ester der Sulfobernsteinsäure oder der Sulfosuccinate der allgemeinen Formel (III), 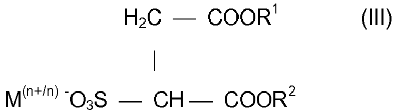 in der M^{(n+/n)} für n = 1 ein Wasserstoffatom, ein Alkalimetallkation, eine Ammoniumgruppe oder das Kation einer ammonium-organischen Base und für n = 2 ein Erdalkalimetallkation darstellt und R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel R-(O-CH₂-CH₂)ₓ-OSO₃H, in der R eine bevorzugt lineare Alkylgruppe mit 8 bis 30 C-Atomen und x = 0 oder 1 - 12 ist,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglycolester,
- Monoglyceridsulfate und Monoglyceridethersulfate.

Bevorzugte anionische Tenside sind Acylglutamate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Zitronensäure oder Bernsteinsäure bzw. der Salze dieser Säuren mit alkylierter Glucose, insbesondere die Produkte mit der INCI-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.

Unabhängig von der Art der eingesetzten Aniontenside sind erfindungsgemäße Haarbehandlungsmittel bevorzugt, die - bezogen auf ihr Gewicht - 2,5 bis 35 Gew.-%, vorzugsweise 5 bis 30 Gew.-%, weiter bevorzugt 7,5 bis 27,5 Gew.-%, noch weiter bevorzugt 10 bis 25 Gew.-% und insbesondere 12,5 bis 22,5 Gew.-% anionische(s) Tensid(e) enthalten.

Ganz besonders bevorzugte erfindungsgemäße Mittel enthalten Fettalkoholsulfate und/oder Fettalkoholethersulfate. Demnach sind erfindungsgemäße Haarbehandlungsmittel, die dadurch gekennzeichnet sind, daß sie als anionisches Tensid - bezogen auf ihr Gewicht - 0,1 bis 20 Gew.-%, vorzugsweise 0,25 bis 17,5 Gew.-% und insbesondere 2 bis 15 Gew.-% Fettalkoholsulfate der Formel

**H₃C-(CH₂)ₙ-OSO₃" M⁺**

enthalten, in der n für Werte von 5 bis 21, vorzugsweise von 7 bis 19, besonders bevorzugt von 9 bis 17 und insbesondere von 11 bis 13 steht, und M für ein Kation aus der Gruppe Na⁺, K⁺ NH₄⁺, ½ Mg²⁺, ½ Zn²⁺, vorzugsweise für Na⁺, steht, bevorzugte Ausführungsformen der vorliegenden Erfindung.

Weiter bevorzugte erfindungsgemäße Haarbehandlungsmittel sind dadurch gekennzeichnet, daß sie als anionisches Tensid - bezogen auf ihr Gewicht - 0,1 bis 20 Gew.-%, vorzugsweise 0,25 bis 17,5 Gew.-% und insbesondere 2 bis 15 Gew.-% Fettalkoholethersulfate der Formel

**H₃C-(CH₂)ₙ-(OCH₂CH₂)ₖOSO₃- M⁺**

enthalten, in der n für Werte von 5 bis 21, vorzugsweise von 7 bis 19, besonders bevorzugt von 9 bis 17 und insbesondere von 11 bis 13 und k für Werte von 1, 2, 3, 4, 5, 6, 7, 8, 9 oder 10, vorzugsweise für 1, 2 oder 3 und insbesondere für 2 stehen, und M für ein Kation aus der Gruppe Na⁺, K⁺ NH₄⁺, ½ Mg²⁺, ½ Zn²⁺, vorzugsweise für Na⁺, steht.

Die erfindungsgemäßen Mittel können zusätzlich zu den anionischen Tensiden weitere Tenside enthalten.

Mit besonderem Vorzug enthalten die erfindungsgemäßen Mittel amphotere(s) Tensid(e). Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Besonders bevorzugte erfindungsgemäße Haarbehandlungsmittel sind dadurch gekennzeichnet, daß sie amphotere(s) Tensid(e) aus den Gruppen der N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren mit jeweils etwa 8 bis 24 C-Atomen in der Alkylgruppe, Alkylaminoessigsäuren mit jeweils etwa 8 bis 24 C-Atomen in der Alkylgruppe, N-Kokosalkylaminopropionat, Kokosacylaminoethylaminopropionat, C₁₂ - C₁₈ - Acylsarcosin, N-Alkyl-N,N-dimethylammonium-glycinate, beispielsweise Kokosalkyl-dimethylammoniumglycinat, N-Acylaminopropyl-N,N-dimethylammoniumglycinate, beispielsweise Kokosacylaminopropyldimethylammoniumglycinat, 2-Alkyl-3-carboxymethyl-3-hydroxyethyl-imidazoline mit jeweils 8 bis 18 C-Atomen in der Alkyl- oder Acylgruppe, Kokosacylaminoethylhydroxyethylcarboxymethylglycinat, der unter der INCI-Bezeichnung Cocamidopropyl Betain bekannten Verbindungen, der unter der INCI-Bezeichnung Disodium Cocoamphodiacetate bekannten Verbindungen enthalten, wobei bevorzugte Mittel das bzw. die amphotere(n) Tensid(e) in Mengen von 0,5 bis 9 Gew.-%, vorzugsweise von 0,75 bis 8 Gew.-% und insbesondere von 1 bis 7,5 Gew.-%, jeweils bezogen auf das gesamte Mittel, enthalten.

Besonders bevorzugte Haarbehandlungsmittel enthalten als amphotere Tenside Betaine der Formel (Bet-I) 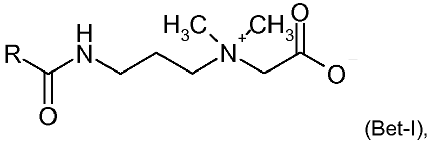 in der R für einen geradkettigen oder verzweigten, gesättigten oder ein- bzw. mehrfach ungesättigten Alkyl- oder Alkenylrest mit 8 bis 24 Kohlenstoffatomen steht.

Diese Tenside werden nach der INCl-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (Bet-I) eingesetzt, die ein Gemisch der folgenden Vertreter sind:
H₃C-(CH₂)₇-C(O)-NH-(CH₂)₃N⁺(CH₃)₂CH₂COO⁻, H₃C-(CH₂)₉-C(O)-NH-(CH₂)₃N⁺(CH₃)₂CH₂COO⁻ , H₃C-(CH₂)₁₁-C(O)-NH-(CH₂)₃N⁺(CH₃)₃₂CH₂COO⁻, H₃C-(CH₂)₁₃-C(O)-NH-(CH₂)₃N⁺(CH₃)₂CH₂COO⁻, H₃C-(CH₂)₁₅-C(O)-NH-(CH₂)₃N⁺(CH₃)₂CH₂COO⁻H₃C-(CH₂)₇-CH=CH-(CH₂)₇-C(O)-NH-(CH₂)₃N⁺(CH₃)₂CH₂COO⁻

Besonders bevorzugt werden Tenside der Formel (Bet-I) innerhalb engerer Mengenbereiche eingesetzt. Hier sind erfindungsgemäße Mittel bevorzugt, die - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (Bet-I) enthalten.

Zusätzlich zu dem bzw. den Amphotensiden der Formel (Bet-I) oder an deren Stelle können die erfindungsgemäßen Haarbehandlungsmittel mit besonderem Vorzug als amphotere Tenside Betaine der Formel (Bet-II) 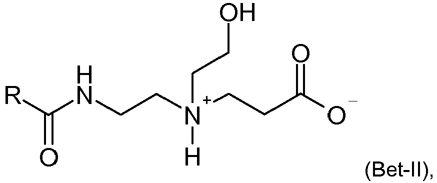 enthalten, in der R für einen geradkettigen oder verzweigten, gesättigten oder ein- bzw. mehrfach ungesättigten Alkyl- oder Alkenylrest mit 8 bis 24 Kohlenstoffatomen steht.

Diese Tenside werden nach der INCI-Nomenklatur als Amphoacetate bezeichnet, wobei die Vertreter, die sich von Kokosfettsäuren ableiten bevorzugt sind und als Cocoamphoactetate bezeichnet werden.

Aus herstellungstechnischen Gründen enthalten Tenside dieses Typs immer auch Betaine der Formel (Bet-IIa) 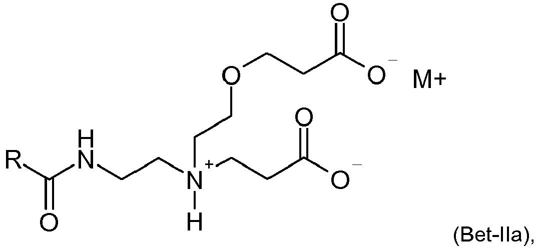 in der R für einen geradkettigen oder verzweigten, gesättigten oder ein- bzw. mehrfach ungesättigten Alkyl- oder Alkenylyrest mit 8 bis 24 Kohlenstoffatomen und M für ein Kation steht. Diese Tenside werden nach der INCI-Nomenklatur als Amphodiacetate bezeichnet, wobei die Vertreter, die sich von Kokosfettsäuren ableiten, bevorzugt sind und als Cocoamphodiactetate bezeichnet werden.

Besonders bevorzugt werden erfindungsgemäß Tenside der Formel (Bet-II) eingesetzt, die ein Gemisch der folgenden Vertreter sind:
H₃C-(CH₂)₇-C(O)-NH-(CH₂)₂NH⁺(CH₂CH₂OH)CH₂CH₂COO-
H₃C-(CH₂)₉-C(O)-NH-(CH₂)₂NH⁺(CH₂CH₂OH)CH₂CH₂COO-
H₃C-(CH₂)₁₁-C(O)-NH-(CH₂)₂NH⁺(CH₂CH₂OH)CH₂CH₂COO⁻
H₃C-(CH₂)₁₃-C(O)-NH-(CH₂)₂NH⁺(CH₂CH₂OH)CH₂CH₂COO⁻
H₃C-(CH₂)₁₅-C(O)-NH-(CH₂)₂NH⁺(CH₂CH₂OH)CH₂CH₂COO⁻
H₃C-(CH₂)₇-CH=CH-(CH₂)₇-C(O)-NH-(CH₂)₂NH⁺(CH₂CH₂OH)CH₂CH₂COO⁻

Besonders bevorzugt werden Tenside der Formel (Bet-II) innerhalb engerer Mengenbereiche eingesetzt. Hier sind erfindungsgemäße Mittel bevorzugt, die - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (Bet-II) enthalten.

Zusammenfassend sind erfindungsgemäße Haarbehandlungsmittel bevorzugt, bei denen der Rest R in den Formeln (Bet-I) und (Bet-II) ausgewählt ist aus H₃C-(CH₂)₇-, H₃C-(CH₂)₉-, H₃C-(CH₂)₁₁-, H₃C-(CH₂)₁₃-, H₃C-(CH₂)₁₅-, H₃C-(CH₂)₇-CH=CH-(CH₂)₇- oder Mischungen aus diesen. Erfindungsgemäß besonders bevorzugte Haarbehandlungsmittel sind dadurch gekennzeichnet, daß sie 1 bis 30 Gew.-%, vorzugsweise 1,5 bis 25 Gew.-%, weiter bevorzugt 2 bis 20 Gew.-%, noch weiter bevorzugt 2,5 bis 15 Gew.-% und insbesondere 3 bis 10 Gew.-% amphotere(s) Tensid(e) enthalten.

Zusätzlich zu den amphoteren Tensiden oder an ihrer Stelle können die erfindungsgemäßen Mittel auch nichtionische Tenside enthalten.

Besonders bevorzugte nichtionische Tenside sind Alkylpolyglycoside. Alkylpolyglycoside (APG) sind nichtionische Tenside, die vollständig aus nachwachsenden Rohstoffen (Zuckerbausteine, vorwiegend Glucose z.B. aus Maisstärke und Fettalkohol z.B. aus Kokosöl) hergestellt werden. Alkylpolyglycoside sind durch sauer katalysierte Reaktion (Fischer-Reaktion) von Zuckern, insbesondere Glucose (oder Stärke) oder von Butylglycosiden mit Fettalkoholen zugänglich.

Dabei entstehen komplexe Gemische aus Alkylmonoglucosid (Alkyl-α-D- und -β-D-glucopyranosid sowie geringe Anteile -glucofuranosid), Alkyldiglucosiden (-isomaltoside, -maltoside etc.) und Alkyloligoglucosiden (-maltotrioside, -tetraoside etc.). Der durchschnittliche Polymerisationsgrad kommerzieller Produkte, deren Alkyl-Reste im Bereich C8-C16 liegen, beträgt 1,2-1,5.

Erfindungsgemäß bevorzugt werden Alkylpolyglycoside entsprechend der allgemeinen Formel RO-(Z)ₓ eingesetzt, wobei R für Alkyl, Z für Zucker sowie x für die Anzahl der Zuckereinheiten steht. Besonders bevorzugt sind solche Alkylpolyglycoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen
besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose. Demnach sind bevorzugte erfindungsgemäße Haarbehandlungsmittel dadurch gekennzeichnet, daß sie- bezogen auf ihr Gewicht - 0,1 bis 20 Gew.-%, vorzugsweise 1 bis 10 Gew.-% und insbesondere 2 bis 8 Gew.-% Alkylpolyglycosid(e) der Formel

**H₃C-(CH₂)ₙ-O-(Z)ₓ**

enthalten, in der n für Werte von 5 bis 21, vorzugsweise von 7 bis 19, besonders bevorzugt von 9 bis 17 und insbesondere von 11 bis 13 und k für Werte von 1,1 bis 1,8, vorzugsweise von 1,2 bis 1,5 stehen, und Z für einen Zuckerbaustein aus der Gruppe Glucose, Fructose, Galactose, Arabinose, Ribose, Xylose, Lyxose, Allose, Altrose, Mannose, Gulose, Idose, Talose und Sucrose steht.

Glucose ist ein besonders bevorzugter Zuckerbaustein (Z), so daß bevorzugte erfindungsgemäße Haarbehandlungsmittel dadurch gekennzeichnet sind, daß sie- bezogen auf ihr Gewicht - 0,1 bis 15 Gew.-%, vorzugsweise 1 bis 10 Gew.-% und insbesondere 2 bis 8 Gew.-% Alkylpolyglucosid(e) der Formel 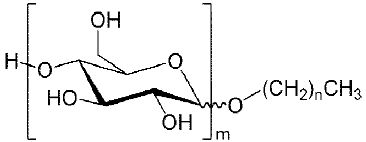 enthalten, in der n für Werte von 5 bis 21, vorzugsweise von 7 bis 19, besonders bevorzugt von 9 bis 17 und insbesondere von 11 bis 13 und m für Werte von 1,1 bis 1,8, vorzugsweise von 1,2 bis 1,5 stehen.

Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.

Ganz besonders bevorzugte Alkypolyglucoside sind solche, deren Alkylrest ein Laurylrest ist. Bei Substanzgemischen aus nativen Quellen sind solche Quellen bevorzugt, die einen hohen Anteil an C12-Fettsäuren haben, insbesondere Cocosfettsäuren. Demnach sind besonders bevorzugte erfindungsgemäße Haarbehandlungsmittel dadurch gekennzeichnet, daß sie- bezogen auf ihr Gewicht - 0,1 bis 15 Gew.-%, vorzugsweise 1 bis 10 Gew.-% und insbesondere 2 bis 8 Gew.-% Alkylpolyglucosid(e) der Formel 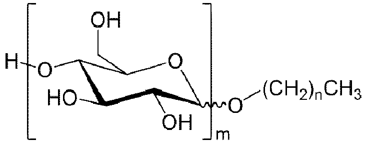 enthalten, in der n für den Wert 11 m für Werte von 1,1 bis 1,8, vorzugsweise von 1,2 bis 1,5 stehen.

Zusammenfassend sind bevorzugte erfindungsgemäße Haarbehandlungsmittel sind dadurch gekennzeichnet, daß sie 5 bis 30 Gew.-%, vorzugsweise 6 bis 25 Gew.-%, weiter bevorzugt 7 bis 20 Gew.-%, noch weiter bevorzugt 8 bis 15 Gew.-% und insbesondere 10 bis 12,5 Gew.-% amphotere(s) und/oder nichtionische Tensid(e) enthalten.

Als zweiten wesentlichen Inhaltsstoff enthalten die erfindungsgemäßen Mittel 0,1 bis 30 Gew.-% mindestens einer Verbindung der allgemeinen Formel (I). In bevorzugten erfindungsgemäßen Mitteln werden die Verbindungen der Formel (I) innerhalb engerer Mengenbereiche eingesetzt, so daß bevorzugte erfindungsgemäße Haarbehandlungsmittel dadurch gekennzeichnet sind, daß sie 0,25 bis 25 Gew.-%, vorzugsweise 0,5 bis 20 Gew.-%, weiter bevorzugt 1 bis 15 Gew.-%, noch weiter bevorzugt 1,5 bis 10 Gew.-% und insbesondere 2 bis 5 Gew.-% mindestens einer Verbindung der allgemeinen Formel (I) enthalten.

In Formel (I) stehen m und n unabhängig voneinander für ganze Zahlen zwischen 5 und 40, wobei die Summe von n + m ≥ 38 ist. Besonders bevorzugte Verbindungen der Formel (I) weisen als Indices n und m die im Prioritätsdokument auf den Seiten 12 bis 24 genannten 926 Paarungen auf.

Bevorzugt gilt die Gleichung n + 4 ≥ m ≥ n -4, d.h. die Acylreste variieren in ihrer Länge um maximal 4 C-Atome voneinander. Ganz besonders bevorzugt gilt die Gleichung n + 2 ≥ m ≥ n -2, d.h. die Acylreste variieren in ihrer Länge um maximal 2 C-Atome voneinander. In solchen besonders bevorzugten Verbindungen der Formel (I) gelten die im Prioritätsdokument auf den Seiten 24 bis 26 genannten Werte für n und m.:
Ganz besonders bevorzugt gilt in Formel (I) n = m, so daß die äußerst bevorzugten Vertreter der Formel (I) die folgenden Verbindungen sind: n = m = 19, n = m = 20, n = m = 21, n = m = 22, n = m = 23, n = m = 24, n = m = 25, n = m = 26, n = m = 27, n = m = 28, n = m = 29, n = m = 30, n = m = 31, n = m = 32, n = m= 33, n = m = 34, n = m = 35, n = m = 36, n = m = 37, n = m = 38, n = m = 39, n = m = 40.

Insbesondere bevorzugt sind die Verbindungen der Formel (I), in denen n = m = 20 gilt.

Die Reste R und R' sind unabhängig voneinander ausgewählt aus -H oder -CH₃. In bevorzugten Verbindungen der Formel (I) gilt R = R', so daß vzw entweder PEG- oder PPG-Diesterquats eingesetzt werden. Ganz besonders bevorzugt gilt R = R' = -CH₃.

Als physiologisch verträgliche Anion X⁻ kommen Halogenide wie Chlorid, Bromid oder lodid in Frage, aber auch Toluolsulfonat, Methosulfat usw.. Ein besonders bevorzugtes Anion ist Methosulfat, so daß bevorzugte erfindungsgemäße Haarbehandlungsmittel 0,25 bis 25 Gew.-%, vorzugsweise 0,5 bis 20 Gew.-%, weiter bevorzugt 1 bis 15 Gew.-%, noch weiter bevorzugt 1,5 bis 10 Gew.-% und insbesondere 2 bis 5 Gew.-% mindestens einer Verbindung der allgemeinen Formel (la) 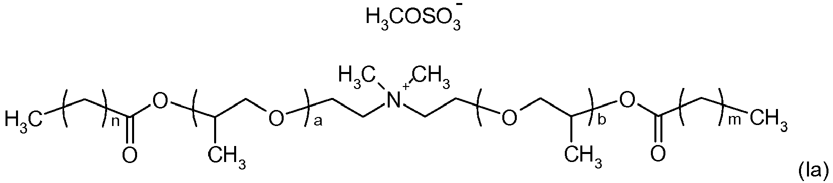 enthalten, in der
n und m unabhängig voneinander für ganze Zahlen zwischen 5 und 40 stehen, mit der Maßgabe, daß n + m ≥ 38 ist;
a und b unabhängig voneinander für 1, 2, 3, 4 oder 5 stehen.

Auch für die Formel (la) gilt das weiter oben für die Indices n und m Gesagte. Eine insbesondere bevorzugte Verbindung der Formel (la) läßt sich demnach durch die Formel (Ia-1) beschreiben:

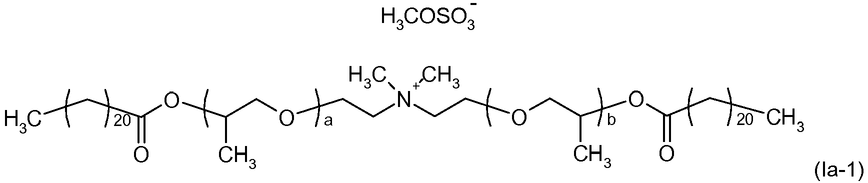

Vorzugsweise stehen die Indices a und b unabhängig voneinander für 1, 2, 3, 4 oder 5. Weiter bevorzugt gilt hierbei die Gleichung a + 2 ≥ b ≥ a -2. In solchen besonders bevorzugten Verbindungen der Formel (I) gilt: a = 1 und b = 1, a = 1 und b = 2, a = 1 und b = 3, a = 2 und b = 1, a = 2 und b = 2, a = 2 und b = 3, a = 2 und b = 4, a = 3 und b = 1, a = 3 und b = 2, a = 3 und b = 3, a = 3 und b = 4, a = 3 und b = 5, a = 4 und b = 2, a = 4 und b = 3, a = 4 und b = 4, a = 4 und b = 5, a = 5 und b = 3, a = 5 und b = 4, a = 5 und b = 5. Ganz besonders bevorzugt gilt a = b, so daß die fünf Verbindungen mit a = b = 1 bzw. a = b = 2 bzw. a = b = 3 bzw. a = b = 4 bzw. a = b = 5 besonders bevorzugt sind.

Bezogen auf die besonders bevorzugte Formel (la-1) sind die folgenden Verbindungen ganz besonders bevorzugt:

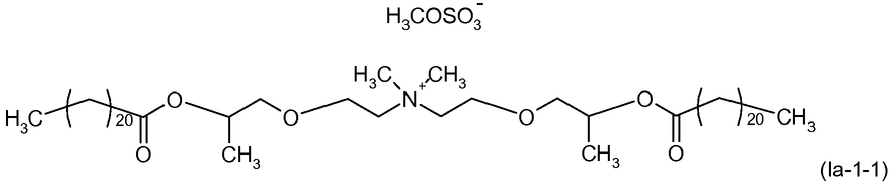

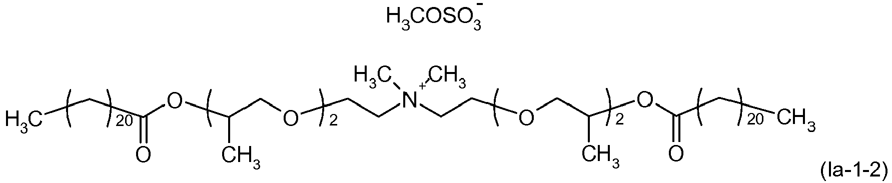

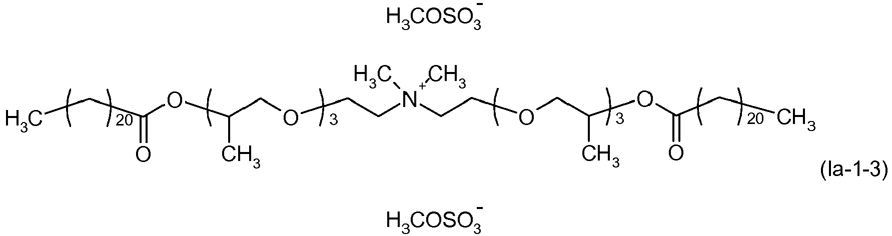

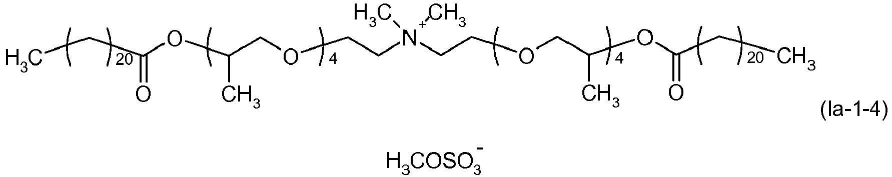

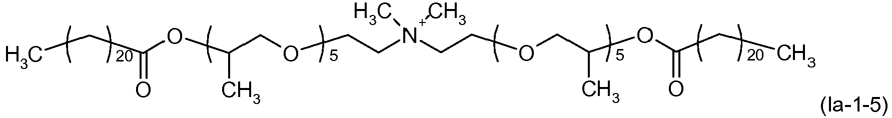

Insbesondere bevorzugt sind Verbindungen der Formel (la-1-3) (nachfolgend als (Ib) bezeichnet), da diese in der erfindungsgemäßen Kombination besonders ausgeprägte Effekte liefern. Erfindungsgemäß bevorzugte Haarbehandlungsmittel sind daher dadurch gekennzeichnet, daß sie 0,5 bis 20 Gew.-%, vorzugsweise 0,75 bis 15 Gew.-%, weiter bevorzugt 1 bis 10 Gew.-%, noch weiter bevorzugt 1,5 bis 7,5 Gew.-% und insbesondere 2 bis 4,5 Gew.-% mindestens einer Verbindung der allgemeinen Formel (Ib) 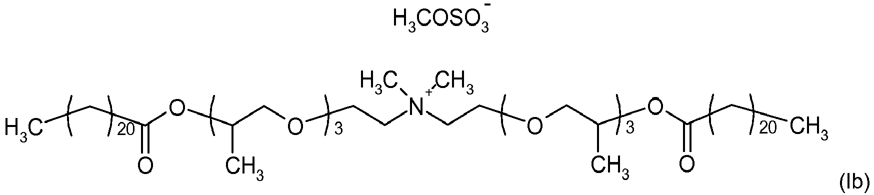 enthalten.

Es hat sich gezeigt, daß die Pflegeeffekte der erfindungsgemäßen Mittel weiter gesteigert und insbesondere die Stabilität der Mittel weiter verbessert werden kann, wenn die Mittel zusätzlich zu der bzw. den Verbindung(en) der Formel (I) bestimmte acylierte Diamine enthalten. Erfindungsgemäße bevorzugte Haarbehandlungsmittel sind daher dadurch gekennzeichnet, daß sie zusätzlich 0,1 bis 10 Gew.-% mindestens einer Verbindung der Formel (II) enthalten 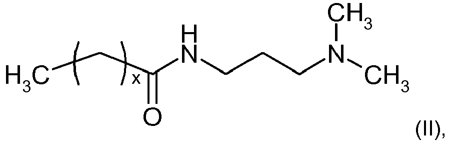 in der x für 18, 19, 20, 21, 22, 23 oder 24 steht. Verbindungen der Formel (II) mit n = 20 sind dabei besonders bevorzugt, so daß äußerst bevorzugte erfindungsgemäße Haarbehandlungsmittel
a) 0,5 bis 20 Gew.-%, vorzugsweise 0,75 bis 15 Gew.-%, weiter bevorzugt 1 bis 10 Gew.-%, noch weiter bevorzugt 1,5 bis 7,5 Gew.-% und insbesondere 2 bis 4,5 Gew.-% mindestens einer Verbindung der allgemeinen Formel (Ib) 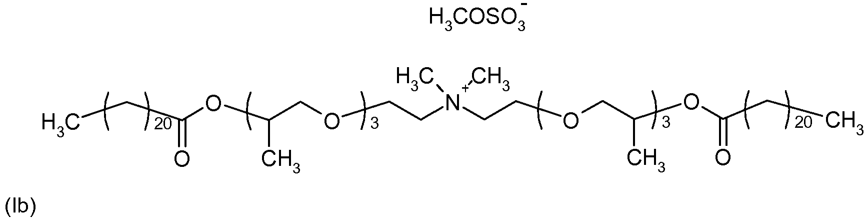
b) 0,1 bis 10 Gew.-% vorzugsweise 0,15 bis 5 Gew.-%, weiter bevorzugt 0,2 bis 2,5 Gew.-%, noch weiter bevorzugt 0,25 bis 1,5 Gew.-% und insbesondere 0,3 bis 1 Gew.-% mindestens einer Verbindung der Formel (IIa) 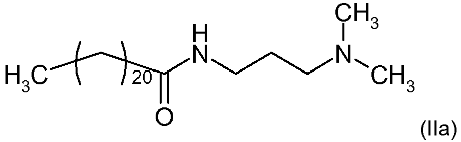 enthalten.

Besonders bevorzugte erfindungsgemäße Haarbehandlungsmittel enthalten
- 2 bis 5 Gew.-% mindestens einer Verbindung der allgemeinen Formel (Ib)
- 0,1 bis 1 Gew.-% mindestens einer Verbindung der Formel (IIa)
- 12,5 bis 22,5 Gew.-% anionische(s) Tensid(e).

Ebenfalls besonders bevorzugte erfindungsgemäße Haarbehandlungsmittel enthalten
- 2 bis 5 Gew.-% mindestens einer Verbindung der allgemeinen Formel (Ib)
- 0,1 bis 1 Gew.-% mindestens einer Verbindung der Formel (IIa)
- 2 bis 15 Gew.-% Fettalkoholsulfate der Formel **H₃C-(CH₂)ₙ-OSO₃⁻ M⁺**

Ebenfalls besonders bevorzugte erfindungsgemäße Haarbehandlungsmittel enthalten
- 2 bis 5 Gew.-% mindestens einer Verbindung der allgemeinen Formel (Ib)
- 0,1 bis 1 Gew.-% mindestens einer Verbindung der Formel (IIa)
- 2 bis 15 Gew.-% Fettalkoholethersulfate der Formel **H₃C-(CH₂)ₙ-(OCH₂CH₂)ₖ-OSO₃⁻ M⁺**

Ebenfalls besonders bevorzugte erfindungsgemäße Haarbehandlungsmittel enthalten
- 2 bis 5 Gew.-% mindestens einer Verbindung der allgemeinen Formel (Ib)
- 0,1 bis 1 Gew.-% mindestens einer Verbindung der Formel (IIa)
- 3 bis 10 Gew.-% amphotere(s) Tensid(e).

Ebenfalls besonders bevorzugte erfindungsgemäße Haarbehandlungsmittel enthalten
- 2 bis 5 Gew.-% mindestens einer Verbindung der allgemeinen Formel (Ib)
- 0,1 bis 1 Gew.-% mindestens einer Verbindung der Formel (IIa)
- 3 bis 10 Gew.-% Betaine der Formel (Bet-I).

Ebenfalls besonders bevorzugte erfindungsgemäße Haarbehandlungsmittel enthalten
- 2 bis 5 Gew.-% mindestens einer Verbindung der allgemeinen Formel (Ib)
- 0,1 bis 1 Gew.-% mindestens einer Verbindung der Formel (IIa)
- 3 bis 10 Gew.-% Betaine der Formel (Bet-II).

Ebenfalls besonders bevorzugte erfindungsgemäße Haarbehandlungsmittel enthalten
- 2 bis 5 Gew.-% mindestens einer Verbindung der allgemeinen Formel (Ib)
- 0,1 bis 1 Gew.-% mindestens einer Verbindung der Formel (IIa)
- 12,5 bis 22,5 Gew.-% nichtionische(s) Tensid(e).

Die Pflegeffekte der erfindungsgemäßen Mittel lassen sich noch weiter verstärken, indem bestimmte Pflegestoffe eingesetzt werden. Vorzugsweise werden diese aus bestimmten Gruppen an sich bekannter Pflegestoffe ausgewählt, da diese Pflegestoffe formulierungstechnisch und vom Pflegeffekt hervorragend mit der erfindungsgemäß eingesetzten Kombination harmonieren. Erfindungsgemäß bevorzugte Haarbehandlungsmittel sind dadurch gekennzeichnet, daß sie zusätzlich Pflegestoff(e) - bezogen auf ihr Gewicht - in Mengen von 0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% enthalten, wobei bevorzugte Pflegstoff(e) ausgewählt sind aus der Gruppe
i. L-Carnitin und/oder seiner Salze;
ii. Taurin und/oder seiner Salze;
iii. Niacinamid;
iv. Ubichinon
v. Ectoin;

L-Carnitin (IUPAC-Name(R)-(3-Carboxy-2-hydroxypropyl)- N,N,N-trimethylammoniumhydroxid), ist eine natürlich vorkommende, vitaminähnliche Substanz. Es spielt eine essentielle Rolle im Energiestoffwechsel menschlicher, tierischer und pflanzlicher Zellen. L-Carnitin kann über verschiedene Wege im industriellen Maßstab gewonnen werden. Beispielsweise über einen, die körpereigene Biosynthese imitierenden, biotechnologischen Prozess: In großen Fermentationsbehältern wird dabei die Vorstufe von L-Carnitin (y-Butyrobetain) mit Hilfe von gramnegativen Bakterien (Rhizobien) in L-Carnitin umgesetzt.

Als Betain kann L-Carnitin Additionsverbindungen und Doppelsalze bilden. Erfindungsgemäß bevorzugte L-Carnitinderivate sind insbesondere ausgewählt aus Acetyl-L-Carnitin, L-Carnitin-Fumarat, L-Carnitin-Citrat, Lauroyl-L-Carnitin und besonderes bevorzugt L-Carnitin-Tartrat. Die genannten L-Carnitin-Verbindungen sind beispielsweise von der Firma Lonza GmbH (Wuppertal, Deutschland) erhältlich.

Erfindungsgemäße bevorzugte Haarbehandlungsmittel sind dadurch gekennzeichnet, daß sie - bezogen auf ihr Gewicht -0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% L-Carnitin oder L-Carnitinderivate enthalten, wobei bevorzugte L-Carnitinderivate ausgewählt sind aus Acetyl-L-Carnitin, L-Carnitin-Fumarat, L-Carnitin-Citrat, Lauroyl- L-Carnitin und insbesondere L-Carnitin-Tartrat.

Ein weiterer, bevorzugter einsetzbarer Pflegestoff, der aktivierende Eigenschaften besitzt, ist das Taurin. Erfindungsgemäß bevorzugte Haarbehandlungsmittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).

Eine weitere bevorzugte Gruppe von Pflegestoffen in den erfindungsgemäßen Mitteln sind Vitamine, Provitamine oder Vitaminvorstufen. Diese werden nachfolgend beschrieben:
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt (siehe weiter unten). Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05- 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäße Haarbehandlungsmittel bevorzugt, die - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel -2,4-Dihydroxy-N-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Es hat sich gezeigt, daß bestimmte Chinone eine besondere Eignung als Pflegestoff besitzen. Als weiteren Pflegestoff können die erfindungsgemäßen Mittel daher 0,0001 bis 5 Gew.-% mindestens eines Biochinons der Formel (Ubi) 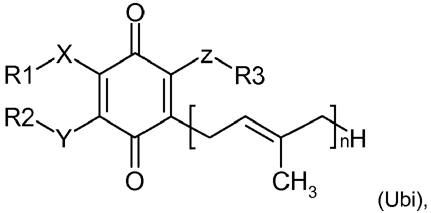 enthalten in der
- X, Y, Z: stehen unabhängig voneinander für -O- oder -NH- oder NR⁴- oder eine chemische Bindung
- R¹, R², R³: stehen unabhängig voneinander für ein Wasserstoffatom oder eine gegebenenfalls substituierte Arylgruppe oder eine gegebenenfalls substituierte (C₁-C₆)-Alkylgruppe oder eine Hydroxyalkylgruppe oder eine Polyhydroxyalkylgruppe oder eine gegebenenfalls substituierte (C₁-C₆)-Alkylengruppe, oder einen (C₁-C₆)-Acylrest, wobei bevorzugte Reste unabhängig voneinander ausgewählt sind aus -H, - CH₃, -CH₂CH₃, -(CH₂)₂CH₂, -CH(CH₃)₂, -(CH₂)₃CH₃, -CH(CH₃)CH₂CH₃, - CH₂CH(CH₃)₂, -C(CH₃)₃
- R⁴: steht für -CH₃, -CH₂CH₃, -(CH₂)₂CH₂, -CH(CH₃)₂, - (CH₂)₃CH₃, -CH(CH₃)CH₂CH₃, -CH₂CH(CH₃)₂, -C(CH₃)₃
- n: steht für Werte von 1 bis 20, vorzugsweise von 2 bis 15 und insbesondere für 5, 6, 7, 8, 9, 10.

Besonders bevorzugte erfindungsgemäße Haarbehandlungsmittel sind dadurch gekennzeichnet, daß sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 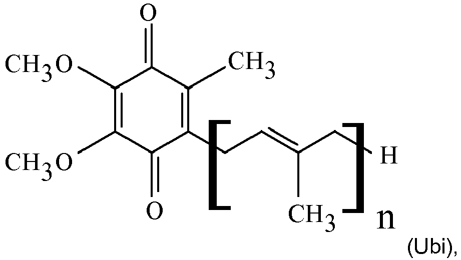 in der n für die Werte = 6, 7, 8, 9 oder 10, besonders bevorzugt für 10 (Coenzym Q10) steht.

Alternativ zu den besonders bevorzugten Ubichinonen oder zusätzlich zu ihnen können die erfindungsgemäßen Mittel auch Plastochinone enthalten. Hier sind bevorzugte erfindungsgemäße Mittel dadurch gekennzeichnet, daß sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons der Formel (Ubi-b) enthalten 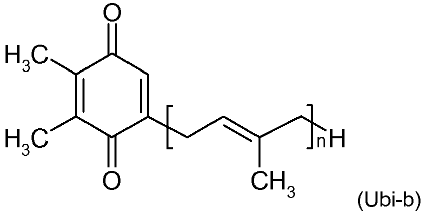 in der n für Werte von 1 bis 20, vorzugsweise von 2 bis 15 und insbesondere für 5, 6, 7, 8, 9, 10 steht, wobei besonders bevorzugt Mittel Plastochinon PQ-9 enthalten.

Als weiteren Pflege-Enhacer können die erfindungsgemäßen Mittel Ectoin enthalten. Ectoin ((4S)-2-Methyl-1,4,5,6-Tetrahydropyrimidin-4-Carbonsäure) ist ein zur Gruppe der kompatiblen Solute gehörender Naturstoff. Die stark wasserbindende niedermolekulare organische Verbindung tritt in halophilen Bakterien auf und ermöglicht diesen extremophilen Organismen unter Stressbedingungen zu überleben. Erfindungsgemäß bevorzugte Haarbehandlungsmittel sind dadurch gekennzeichnet, daß sie - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidincarbonsäure (Hydroxyectoin) sowie die physiologisch verträglichen Salze dieser Verbindung, enthalten.

Zur Verbesserung der Elastizität und Festigung der inneren Struktur der mit erfindungsgemäßen Mitteln behandelter Haare können die erfindungsgemäßen Mittel Purin und/oder Purinderivate als Pflegestoff enthalten. Insbesondere die Kombination von Purin und/oder Purinderivaten mit Ubichinonen und/oder Plastochinonen als Pflegestoff führt dazu, daß die mit entsprechenden Mitteln behandelten Haare unter anderem höhere Meßwerte bei der Differenzthermoanalyse und verbesserte Naß- und Trockenkämmbarkeiten zeigen.

Purin (7H-Imidazo[4,5-d]pyrimidin) kommt frei in der Natur nicht vor, bildet jedoch den Grundkörper der Purine. Purine ihrerseits sind eine Gruppe wichtiger, in der Natur weit verbreiteter und an menschlichen, tierischen, pflanzlichen und mikrobiellen Stoffwechselvorgängen beteiligter Verbindungen, die sich vom Grundkörper durch Substitution mit OH, NH₂, SH in 2-, 6- und 8-Stellung und/oder mit CH₃ in 1-, 3-, 7-Stellung ableiten. Purin kann beispielsweise aus Aminoacetonitril und Formamid hergestellt werden. Purine und Purinderivate werden oft aus Naturstoffen isoliert, sind aber auch auf vielen Wegen synthetisch zugänglich.

Bevorzugte erfindungsgemäße Mittel enthalten Purin und/oder Purinderivate in engeren Mengenbereichen. Hier sind erfindungsgemäß bevorzugte kosmetische Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.

Unter Purin, den Purinen und den Purinderivaten sind erfindungsgemäß einige Vertreter besonders bevorzugt. Erfindungsgemäß bevorzugte Haarbehandlungsmittel sind dadurch gekennzeichnet, daß sie als Pflegestoff - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthält, wobei bevorzugte Mittel Purin und/oder Purinderivat(e) der Formel (Pur-I) enthalten 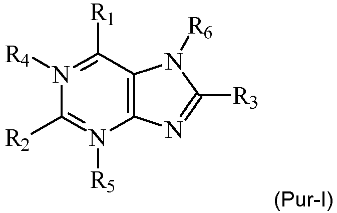 in der die Reste R¹, R² und R³ unabhängig voneinander ausgewählt sind aus -H, - OH, NH₂, -SH und die Reste R⁴, R⁵ und R⁶ unabhängig voneinander ausgewählt sind aus -H, -CH₃ und -CH₂-CH₃, wobei folgende Verbindungen bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H).

Es ist weiterhin vorteilhaft, Purin bzw. Purinderivate und Biochinone in einem bestimmten Verhältnis zueinander einzusetzen. Hier sind erfindungsgemäße Mittel bevorzugt, bei denen das Gewichtsverhältnis von Purin(derivat(en)) und Biochinon(en) 10:1 bis 1:100, vorzugsweise 5:1 bis 1:50, besonders bevorzugt 2:1 bis 1:20 und insbesondere 1:1 bis 1:10 beträgt.

Wie bereits erwähnt, ist Coffein ein besonders bevorzugtes Purinderivat, und das Coenzym Q10 ist ein besonders bevorzugtes Biochinon. Besonders bevorzugte erfindungsgemäße Mittel sind daher dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Coffein und 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% Coenzym Q10 enthalten.

Als Pflegestoff können die erfindungsgemäßen Mittel auch Flavonoide enthalten. Die Flavonoide sind eine Gruppe von wasserlöslichen Pflanzenfarbstoffen und spielen eine wichtige Rolle im Stoffwechsel vieler Pflanzen. Sie gehören zusammen mit den Phenolsäuren zu den Polyphenolen. Es sind weit über 6500 unterschiedliche Flavonoide bekannt, die sich in Flavonole, Flavone, Flavanone, Isoflavonoide und Anthocyane einteilen lassen.

Erfindungsgemäß können Flavonoide aus allen sechs Gruppen eingesetzt werden, wobei bestimmte Vertreter aus den einzelnen Gruppen als Pflegestoff wegen ihrer besonders intensiven Wirkung bevorzugt sind. Bevorzugte Flavonole sind Quercetin, Rutin, Kaempferol, Myricetin, Isorhamnetin, bevorzugte Flavanole sind Catechin, Gallocatechin, Epicatechin, Epigallocatechingallat , Theaflavin, Thearubigin, bevorzugte Flavone sind Luteolin, Apigenin, Morin, bevorzugte Flavanone sind Hesperetin, Naringenin, Eriodictyol, bevorzugte Isoflavonoide sind Genistein, Daidzein, und bevorzugte Anthocyanidine (Anthocyane) sind Cyanidin, Delphinidin, Malvidin, Pelargonidin, Peonidin, Petunidin.

Erfindungsgemäß besonders bevorzugte Haarbehandlungsmittel sind dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Flavonoide, insbesondere Flavonole, besonders bevorzugt 3,3',4',5,7-Pentahydroxyflavon (Quercetin) und/oder 3,3',4',5,7-Pentahydroxyflavon-3-O-rutinosid (Rutin), enthalten.

Bevorzugt ist auch der Einsatz von Bisabolol und/oder Bisabololoxiden als Pflegestoff in den erfindungsgemäßen Mitteln. Hier sind erfindungsgemäße Haarbehandlungsmittel bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol enthalten.

Auch Creatin eignet sich erfindungsgemäß als Pflegestoff. Creatin (3-Methylguanidinoessigsäure) ist eine organische Säure, die in Wirbeltieren u. a. zur Versorgung der Muskeln mit Energie beiträgt. Kreatin wird in der Niere, der Leber und in der Bauchspeicheldrüse synthetisiert. Sie leitet sich formal von den Aminosäuren Glycin und Arginin ab und ist zu 95 % im Skelettmuskel vorhanden. Besonders bevorzugte erfindungsgemäße Haarbehandlungsmittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% N-Methylguanidino-essigsäure (Creatin).

Die erfindungsgemäßen Mittel können zusätzlich zu den vorstehend genannten Inhaltsstoffen und optionalen weiteren Inhaltsstoffen weitere Stoffe enthalten, die Haarausfall verhindern, lindern oder heilen. Insbesondere ist ein Gehalt an haarwurzelstabilisierenden Wirkstoffen vorteilhaft. Diese Stoffe werden nachstehend beschrieben:
Propecia (Finasterid) ist das zur Zeit einzige Präparat, das weltweit zugelassen ist und für das in zahlreichen Studien eine Wirksamkeit und Verträglichkeit nachgewiesen wurde. Propecia bewirkt, daß sich weniger DHT aus Testosteron bilden kann.

Minoxidil ist mit oder ohne ergänzende Zusatzstoffe das wohl älteste nachweislich wirkende Haarwuchsmittel. Zur Behandlung von Haarausfall darf es nur zur äußeren Anwendung verwendet werden. Es gibt Haarwasser, die 2%-5% Minoxidil enthalten, außerdem Gels mit bis zu 15% Minoxidil. Die Wirksamkeit nimmt mit der Dosierung zu, in Haarwassern ist Minoxidil jedoch nur bis zu 5% Anteil löslich. In vielen Ländern sind Haarwasser mit bis zu 2% Minoxidilgehalt verschreibungsfrei erhältlich.

Zur Bekämpfung der hormonellen Einflüße auf die Haarfollikel kann zur äußeren Anwendung Spironolactone in Form von Haarwasser und in Kombination mit Minoxidil angewandt werden. Spironolactone wirkt als Androgen-Rezeptor-Blocker, dh. die Bindung von DHT an die Haarfollikel wird verhindert.

Zusammenfassend sind erfindungsgemäße Haarbehandlungsmittel bevorzugt, die zusätzlich - bezogen auf sein Gewicht - 0,001 bis 5 Gew.-% Haarwurzel-stabilisierende Stoffe, insbesondere Minoxidil und/oder Finasterid und/oder Ketoconazol enthalten.

Zusätzlich zu den Pflegestoffen können di erfindungsgemäßen Mittel weitere Pflegestoffe enthalten. Deren Anwesenheit ist für die Erzielung der erfindungsgemäßen Effekte nicht zwingend erforderlich, doch können weitergehende Effekte, wie ein angenehmer Griff oder eine angenehme Applikationshaptik aus dem Einsatz dieser Pflegestoffe resultieren.

Als weiteren Inhaltsstoff können die erfindungsgemäßen Mittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L*-trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte Haarbehandlungsmittel dadurch gekennzeichnet, daß sie als Pflegestoff - bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanain und/oder Valin und/oder Lysin und/oder Leucin und/oder Threonin enthalten.

Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte Haarbehandlungsmittel dadurch gekennzeichnet, daß sie als Pflegestoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus Monosachhariden, insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose Disacchariden, insbesondere Saccharose und/oder Maltose und/oder Lactose und/oder Trehalose und/oder Cellobiose und/oder Gentiobiose und/oder Isomaltose.

Besonders bevorzugte erfindungsgemäße Mittel enthalten bezogen auf ihr Gewicht
- 0,005 bis 0,015 Gew.-% Coffein und 0,75 bis 1,5 Gew.-% Glucosemonohydrat,
- 0,005 bis 0,015 Gew.-% Coffein und 0,75 bis 1,5 Gew.-% Saccharose,
- 0,005 bis 0,015 Gew.-% Coffein und 0,75 bis 1,5 Gew.-% Fructose.

Wie bereits erwähnt, enthalten bevorzugte erfindungsgemäße Mittel (eine) Aminosäure(n). Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte kosmetische Mittel dadurch gekennzeichnet, daß sie zusätzlich - 0,05 bis 5 Gew.-%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthalten.

Besonders bevorzugte erfindungsgemäße Mittel enthalten bezogen auf ihr Gewicht
- 0,005 bis 0,015 Gew.-% Coffein und 0,75 bis 1,5 Gew.-% Glucosemonohydrat und 0,1 bis 0,25 Gew.-% Glycin,
- 0,005 bis 0,015 Gew.-% Coffein und 0,75 bis 1,5 Gew.-% Saccharose und 0,1 bis 0,25 Gew.-% Glycin,
- 0,005 bis 0,015 Gew.-% Coffein und 0,75 bis 1,5 Gew.-% Fructose und 0,1 bis 0,25 Gew.-% Glycin,
- 0,005 bis 0,015 Gew.-% Coffein und 0,75 bis 1,5 Gew.-% Glucosemonohydrat und 0,1 bis 0,25 Gew.-% Alanin,
- 0,005 bis 0,015 Gew.-% Coffein und 0,75 bis 1,5 Gew.-% Saccharose und 0,1 bis 0,25 Gew.-% Alanin,
- 0,005 bis 0,015 Gew.-% Coffein und 0,75 bis 1,5 Gew.-% Fructose und 0,1 bis 0,25 Gew.-% Alanin,
- 0,005 bis 0,015 Gew.-% Coffein und 0,75 bis 1,5 Gew.-% Glucosemonohydrat und 0,1 bis 0,25 Gew.-% Valin,
- 0,005 bis 0,015 Gew.-% Coffein und 0,75 bis 1,5 Gew.-% Saccharose und 0,1 bis 0,25 Gew.-% Valin,
- 0,005 bis 0,015 Gew.-% Coffein und 0,75 bis 1,5 Gew.-% Fructose und 0,1 bis 0,25 Gew.-% Valin.

Eine besonders bevorzugte Gruppe von Inhaltsstoffen stellen die Silikone dar.

Die Silikone stammen vorzugsweise aus den Gruppen der Dimethicone und/oder der Cylomethicone und/oder der Amodimethicone und/oder der Dimethiconole und/oder der Trisiloxane. Erfindungsgemäß bevorzugte Haarbehandlungsmittel sind dadurch gekennzeichnet, daß sie 0,15 bis 15 Gew.-%, vorzugsweise 0,2 bis 10 Gew.-%, weiter bevorzugt 0,25 bis 7,5 Gew.-%, noch weiter bevorzugt 0,5 bis 5 Gew.-% und insbesondere 0,75 bis 2,5 Gew.-% Silikon(e) aus den Gruppen der Dimethicone und/oder der Cylomethicone und/oder der Amodimethicone und/oder der Dimethiconole enthalten.

Dimethicone können sowohl linear als auch verzweigt als auch cyclisch oder cyclisch und verzweigt sein. Lineare Dimethicone können durch die folgende Strukturformel (Si1) dargestellt werden:

(SiR¹₃) - O - (SiR²₂ - O -)ₓ - (SiR¹₃) (Si1)

Verzweigte Dimethicone können durch die Strukturformel (Si1.1) dargestellt werden:

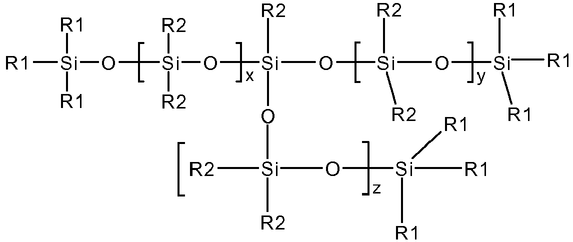

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs. Höchst bevorzugt sind Viskositäten um den Bereich von etwa 60.000 cPs herum. Das Wort "etwa" definiert dabei eine dem Fachmann bei technisch hergestellten Produkten übliche Abweichung von dem genannten Wert im Anschluß an das Wort "etwa". Beispielhaft sei hier auf das Produkt "Dow Corning 200 mit 60000cSt" verwiesen. Selbstverständlich umfasst die erfindungsgemäße Lehre auch, dass die Dimethicone bereits als Emulsion vorliegen können.

Wenn die Dimethicone als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und µm bevorzugtesten 0,01 bis 10 µm. Die Teilchengröße wird dabei nach der Methode der Lichtstreuung bestimmt.

Besonders bevorzugte erfindungsgemäße Haarbehandlungsmittel sind dadurch gekennzeichnet, daß sie 0,15 bis 15 Gew.-%, vorzugsweise 0,2 bis 10 Gew.-%, weiter bevorzugt 0,25 bis 7,5 Gew.-%, noch weiter bevorzugt 0,5 bis 5 Gew.-% und insbesondere 0,75 bis 2,5 Gew.-% mindestens eines Silikons der Formel (Si-I)

(CH₃)₃Si-[O-Si(CH₃)₂]ₓ-O-Si(CH₃)₃ (Si-I),

enthalten, in der x für eine Zahl von 0 bis 100, vorzugsweise von 0 bis 50, weiter bevorzugt von 0 bis 20 und insbesondere 0 bis 10, steht.

Besonders bevorzugte erfindungsgemäße Mittel enthalten ein oder mehrere aminofunktionelle Silikone. Solche Silikone können z.B. durch die Formel (Si-2)

M(RₐQ_{b}SiO_{(4-a-b)/2})x(R_{c}SiO_{(4-c)/2})_{y}M (Si-2)

beschrieben werden, wobei in der obigen Formel
- R: ein Kohlenwasserstoff oder ein Kohlenwasserstoffrest mit 1 bis etwa 6 Kohlenstoffatomen ist,
- Q: ein polarer Rest der allgemeinen Formel -R¹HZ ist, worin
- R¹: eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
- Z: ein organischer, aminofunktioneller Rest ist, der mindestens eine aminofunktionelle Gruppe enthält;
- a: Werte im Bereich von etwa 0 bis etwa 2 annimmt,
- b: Werte im Bereich von etwa 1 bis etwa 3 annimmt,
- a + b: kleiner als oder gleich 3 ist, und
- c: eine Zahl im Bereich von etwa 1 bis etwa 3 ist, und
- x: eine Zahl im Bereich von 1 bis etwa 2.000, vorzugsweise von etwa 3 bis etwa 50 und am bevorzugtesten von etwa 3 bis etwa 25 ist, und
- y: eine Zahl im Bereich von etwa 20 bis etwa 10.000, vorzugsweise von etwa 125 bis etwa 10.000 und am bevorzugtesten von etwa 150 bis etwa 1.000 ist, und
- M: eine geeignete Silikon-Endgruppe ist, wie sie im Stande der Technik bekannt ist, vorzugsweise Trimethylsiloxy.

Nicht einschränkende Beispiele der in Formel (Si-2) durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH ₂-, -CH₂CH₂OCH₂-, -OCH2CHz-, - OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein.

Z ist gemäß Formel (Si-2) ein organischer, aminofunktioneller Rest, enthaltend mindestens eine funktionelle Aminogruppe. Eine mögliche Formel für besagtes Z ist NH(CH₂)_{z}NH₂, worin z eine ganze Zahl von größer gleich 1 ist. Eine andere mögliche Formel für besagtes Z ist -NH(CH₂)_{z}(CH ₂)_{zz}NH, worin sowohl z als auch zz unabhängig voneinander eine ganze Zahl von größer gleich 1 sind, wobei diese Struktur Diamino-Ringstrukturen umfasst, wie Piperazinyl. Besagtes Z ist am bevorzugtesten ein -NHCH₂CH ₂NH₂-Rest. Eine andere mögliche Formel für besagtes Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q gemäß Formel (Si-2) ist am bevorzugtesten ein polarer aminofunktioneller Rest der Formel - CH₂CH₂CH₂NHCH₂CH₂NH₂.

In der Formel (Si-2) nimmt a Werte im Bereich von 0 bis 2 an, b nimmt Werte im Bereich von 2 bis 3 an, a + b ist kleiner als oder gleich 3, und c ist eine Zahl im Bereich von 1 bis 3. Das molare Verhältnis der RₐQ_{b} SiO_{(4-a-b)/2}-Einheiten zu den R_{c}SiO _{(4-c)/2}-Einheiten in Formel (Si-2) liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silikone der obigen Formel (Si-2) eingesetzt, dann können die verschiedenen variablen Substituenten in der obigen Formel bei den verschiedenen Silikonkomponenten, die in der Silikonmischung vorhanden sind, verschieden sein.

Bevorzugte erfindungsgemäße Haarbehandlungsmittel enthalten ein aminofunktionelles Silikon der Formel (Si-3)

R'ₐG₃₋ₐ-Si(OSiG₂)ₙ-(OSiG_{b}R'_{2-b)m}-O-SIG₃₋ₐ-R'ₐ (Si-3),

worin bedeutet:
- G: ist -H, eine Phenylgruppe, -OH, -O-CH₃, -CH₃, -O-CH₂CH₃, -CH₂CH₃, -O-CH₂CH₂CH₃, -CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃ ;
- a: steht für eine Zahl zwischen 0 und 3, insbesondere 0;
- b: steht für eine Zahl zwischen 0 und 1, insbesondere 1,
- m und n: sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R': ist ein monovalenter Rest ausgewählt aus

- Q-N(R")-CH₂-CH₂-N(R")₂
- Q-N(R")₂
- Q-N⁺(R")₃A⁻
- Q-N⁺H(R")₂ A⁻
- Q-N⁺H₂(R")A⁻
- Q-N(R")-CH₂-CH₂-N⁺R"H₂A⁻,
   wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
   R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, lodid oder Methosulfat.

Erfindungsgemäß geeignet sind kationische Silikonöle wie beispielsweise die im Handel erhältliche Dow Corning 929 Emulsion (enthaltend ein hydroxylamino-modifiziertes Silikon, das als Amodimethicone bezeichnet wird), DC 2-2078 (Hersteller Dow Corning, INCI-Bezeichnung: Aminopropyl Phenyl Trimethicone), DC 5-7113 (Hersteller Dow Corning, INCI-Bezeichnung: Silicone Quaternium 16), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Besonders bevorzugte erfindungsgemäße Haarbehandlungsmittel sind dadurch gekennzeichnet, daß sie 0,15 bis 15 Gew.-%, vorzugsweise 0,2 bis 10 Gew.-%, weiter bevorzugt 0,25 bis 7,5 Gew.-%, noch weiter bevorzugt 0,5 bis 5 Gew.-% und insbesondere 0,75 bis 2,5 Gew.-% mindestens eines aminofunktionellen Silikons der Formel (Si-IIa) 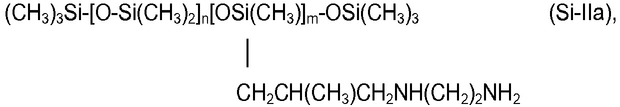 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Weitere besonders bevorzugte Haarbehandlungsmittel sind dadurch gekennzeichnet, daß sie 0,15 bis 15 Gew.-%, vorzugsweise 0,2 bis 10 Gew.-%, weiter bevorzugt 0,25 bis 7,5 Gew.-%, noch weiter bevorzugt 0,5 bis 5 Gew.-% und insbesondere 0,75 bis 2,5 Gew.-% mindestens eines aminofunktionellen Silikons der Formel (Si-IIb) 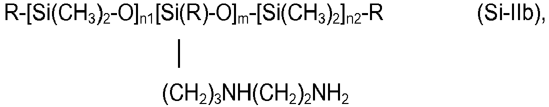 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Mittel, die mindestens ein aminofunktionelles Silikon der Formel (Si-3b) 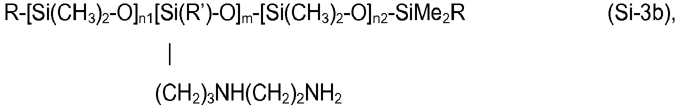 enthalten, worin
- R: für -OH, eine (gegebenenfalls ethoxylierte und/oder propoxylierte) (C₁ bis C₂₀)-Alkoxygruppe oder eine -CH₃-Gruppe steht,
- R': für -OH, eine (C₁ bis C₂₀)-Alkoxygruppe oder eine -CH₃-Gruppe und
- m, n1 und n2: Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich) bezeichnet. Unabhängig davon, welche aminofunktionellen Silikone eingesetzt werden, sind erfindungsgemäße kosmetische oder dermatologische Zubereitungen bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silikons. Sie kann durch Titration ermittelt und auch in der Einheit mg KOH/g angegeben werden.

Erfindungsgemäß bevorzugte Haarbehandlungsmittel sind dadurch gekennzeichnet, dass sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Als Silikon können die erfindungsgemäßen Zusammensetzungen auch mindestens eine Polyammonium-Polysiloxan Verbindung enthalten. Die Polyammonium-Polysiloxan Verbindungen können beispielsweise unter der Handelsbezeichnung Baysilone^{®} von GE Bayer Silicones bezogen werden. Die Produkte mit den Bezeichnungen Baysilone TP 3911, SME 253 und SFE 839 sind dabei bevorzugt. Ganz besonders bevorzugt ist die Verwendung von Baysilone TP 3911 als Wirkkomponente der erfindungsgemäßen Zusammensetzungen.

Die Polyammonium-Polysiloxan Verbindungen werden in dem erfindungsgemäßen Zusammensetzungen vorzugsweise in einer Menge von 0,01 bis 10 Gew.% , vorzugsweise 0,01 bis 7,5, besonders bevorzugt 0,01 bis 5,0 Gew.%, ganz besonders bevorzugt von 0,05 bis 2,5 Gew.% jeweils in Bezug auf die Gesamtzusammensetzung verwendet.

Auch die nach INCI als Cyclomethicone bezeichneten cyclischen Dimethicone sind erfindungsgemäß mit Vorzug einsetzbar. Hier sind erfindungsgemäße Haarbehandlungsmittel bevorzugt, die mindestens ein Silikon der Formel (Si-4) 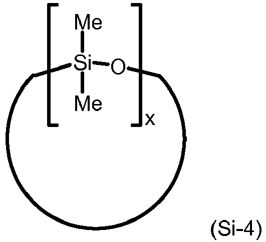 enthalten, in der x für eine Zahl von 3 bis 200, vorzugsweise von 3 bis 10, weiter bevorzugt von 3 bis 7 und insbesondere 3, 4, 5 oder 6, steht.

Die vorstehend beschriebenen Silikone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Erfindungsgemäß ebenfalls bevorzugte Mittel sind dadurch gekennzeichnet, dass sie mindestens ein Silikon der Formel (Si-5)

R₃Si-[O-SiR₂]ₓ-(CH₂)ₙ-[O-SiR₂]_{y}-O-SiR₃ (Si-5),

enthalten, in der R für gleiche oder verschiedene Reste aus der Gruppe -H, - Phenyl, -Benzyl, -CH₂-CH(CH₃)Ph, der C₁₋₂₀-Alkylreste, vorzugsweise -CH₃, - CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Als weitere Silikone neben den Dimethiconen, Dimethiconolen, Amodimethiconen und/oder Cyclomethiconen können wasserlösliche Silikone in den erfindungsgemäßen Zusammensetzungen enthalten sein.

Entsprechende hydrophile Silikone werden beispielsweise aus den Verbindungen der Formeln (Si-6) und/oder (Si-7) ausgewählt. Insbesondere bevorzugte wasserlösliche Tenside auf Silikonbasis sind ausgewählt aus der Gruppe der Dimethiconcopolyole die bevorzugt alkoxyliert, insbesondere polyethoxyliert oder polypropoxyliert sind.

Unter Dimethiconcopolyolen werden erfindungsgemäß bevorzugt Polyoxyalkylen-modifizierte Dimethylpolysiloxane der allgemeinen Formeln (Si-6) oder (Si-7) verstanden: 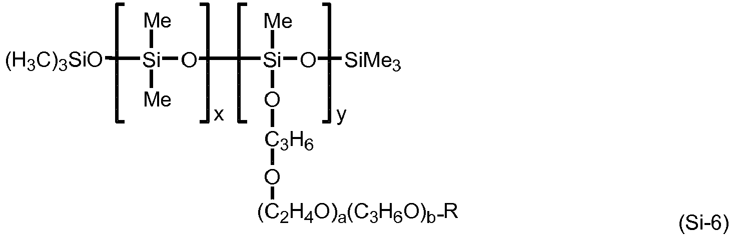 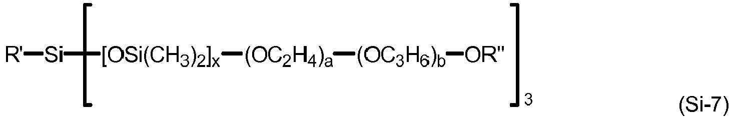 worin - der Rest R steht für ein Wasserstoffatom, eine Alkylgruppe mit 1 bis 12 C-Atomen, eine Alkoxygruppe mit 1 bis 12 C-Atomen oder eine Hydroxylgruppe,
- die Reste R' und R" bedeuten Alkylgruppen mit 1 bis 12 C-Atomen,
- x steht für eine ganze Zahl von 1 bis 100, bevorzugt von 20 bis 30,
- y steht für eine ganze Zahl von 1 bis 20, bevorzugt von 2 bis 10 und
- a und b stehen für ganze Zahlen von 0 bis 50, bevorzugt von 10 bis 30.

Besonders bevorzugte Dimethiconcopolyole im Sinne der Erfindung sind beispielsweise die kommerziell unter dem Handelsnamen SILWET (Union Carbide Corporation) und DOW CORNING (Dow) vertriebenen Produkte. Erfindungsgemäß besonders bevorzugte Dimethiconcopolyole sind Dow Corning 190 und Dow Corning 193 (Dow).

Die Dimethiconcopolyole sind in den erfindungsgemäßen Zusammensetzungen vorzugsweise in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconcopolyol bezogen auf die Zusammensetzung.

Schließlich werden unter den Silikonverbindungen die Dimethiconole (Si8) verstanden. Dimethiconole bilden eine weitere Gruppe der Silikone, welche erfindungsgemäß besonders bevorzugt sind. Die erfindungsgemäßen Dimethiconole können sowohl linear als auch verzweigt als auch cyclisch oder cyclisch und verzweigt sein. Lineare Dimethiconole können durch die folgende Strukturformel (Si8 - I) dargestellt werden:

(SiOHR¹₂)-O-(SiR²₂-O- )x - (SiOHR¹₂) (Si8 - I)

Verzweigte Dimethiconole können durch die Strukturformel (Si8 - II) dargestellt werden:

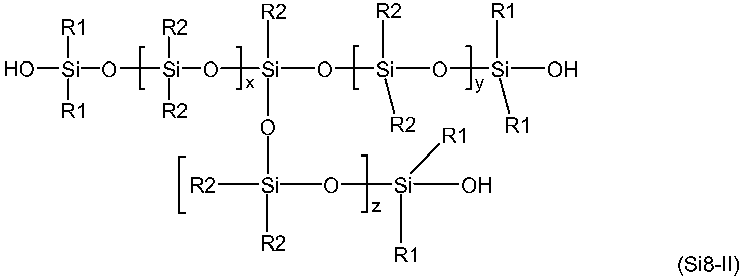

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethiconole liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Wenn die erfindungsgemäßen Dimethiconole als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Als Beispiele für derartige Produkte werden die folgenden Handelsprodukte genannt: Dow Corning 1-1254 Fluid, Dow Corning 2-9023 Fluid, Dow Corning 2-9026 Fluid, X-21-5619 (Shin-Etsu Chemical Co.), Abil OSW 5 (Degussa Care Specialties), ACC DL-9430 Emulsion (Taylor Chemical Company), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Silsoft 148, Silsoft E-50, Silsoft E-623 (alle zuvor genannten Crompton Corporation), SM555, SM2725, SM2765, SM2785 (alle zuvor genannten GE Silicones), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Die Dimethiconole (Si8) sind in den erfindungsgemäßen Zusammensetzungen vorzugsweise in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconol bezogen auf die Zusammensetzung. Wenn eine Mischung aus mindestens zwei Silikonen verwendet wird, so ist diese Mischung in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Silikonmischung bezogen auf die Zusammensetzung enthalten.

Aus ästhetischen Gründen werden "klare" Produkte von Verbrauchern oft bevorzugt. Erfindungsgemäß bevorzugte Haarbehandlungsmittel sind daher dadurch gekennzeichnet, daß sie transparent bzw. transluzent sind.

Unter transparent oder transluzent wird im Rahmen der vorliegenden Erfindung eine Zusammensetzung verstanden, die einen NTU-Wert von unter 100 aufweist. Der NTU-Wert (Nephelometric Turbidity Unit, *Nephelometrischer Trübungswert*; NTU) ist eine in der Wasseraufbereitung verwendete Einheit für Trübungsmessungen in Flüssigkeiten. Sie ist die Einheit einer mit einem kalibriertem Nephelometer gemessenen Trübung einer Flüssigkeit. Weiterhin kann in einer bevorzugten Ausführungsform der Erfindung ein erfindungsgemäßes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Die UV-Filter (I) sind in den erfindungsgemäßen Mitteln üblicherweise in Mengen 0,1-5 Gew.-%, bezogen auf das gesamte Mittel, enthalten. Mengen von 0,4-2,5 Gew.-% sind bevorzugt.

Die erfindungsgemäßen Mittel können weiterhin eine 2-Pyrrolidinon-5-carbonsäure und deren Derivate (J) enthalten. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf das gesamte Mittel, besonders bevorzugt 0,1 bis 5, und insbesondere 0,1 bis 3 Gew.%.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol. Vorteilhaft im Sinne der Erfindung können zusätzlich kurzkettige Carbonsäuren (N) den Wirkstoffkomplex (A) unterstützen. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäß verwendeten Carbonsäuren können selbstverständlich entlang der Kohlenstoffkette oder des Ringgerüstes substituiert sein. Zu den Substituenten der erfindungsgemäß verwendeten Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl-,C2-C8-Hydroxyalkenyl-, Aminomethyl-, C2-C8-Aminoalkyl-, Cyano-, Formyl-, Oxo-, Thioxo-, Hydroxy-, Mercapto-, Amino-, Carboxy- oder Iminogruppen. Bevorzugte Substituenten sind C1-C8-Alkyl-, Hydroxymethyl-, Hydroxy-, Amino- und Carboxygruppen. Besonders bevorzugt sind Substituenten in - Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Ein weiterer Gegenstand der vorliegenden Erfindung ist ein Verfahren zur Behandlung von keratinischen Fasern, insbesondere menschlichen Haaren, bei dem ein erfindungsgemäßes Haarbehandlungsmittel auf keratinische Fasern aufgebracht und dort entweder bis zur nächsten Haarwäsche belassen (sogenanntes "leave-on"-Produkt) oder nach einer Einwirkzeit von 30 bis 900 Sekunden ausgespült (sogenanntes "rinse-off"-Produkt) wird.

Ein weiterer Gegenstand der vorliegenden Erfindung ist daher ein Verfahren zur Haarbehandlung, bei dem ein erfindungsgemäßes Haarbehandlungsmittel auf das Haar aufgebracht und nach einer Einwirkzeit von 5 Sekunden bis 15 Minuten wieder ausgespült wird.

Ein weiterer Gegenstand der vorliegenden Erfindung ist daher ein Verfahren zur Haarbehandlung, bei dem ein erfindungsgemäßes Haarbehandlungsmittel auf das Haar aufgebracht und dort bis zur nächsten Haarwäsche belassen wird.

Bezüglich bevorzugter Ausführungsformen für die erfindungsgemäßen Verfahren gilt mutatis mutandis das zu den erfindungsgemäßen Mitteln Gesagte.

## Patentansprüche

1. Haarbehandlungsmittel, enthaltend - bezogen auf sein Gewicht -
a) 0,1 bis 40 Gew.-% anionische(s) und/oder amphotere(s) und/oder nichtionische(s) Tensid(e),
b) 0,1 bis 30 Gew.-% mindestens einer Verbindung der allgemeinen Formel (I) 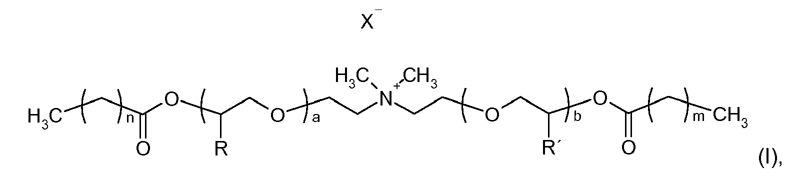 in der
n und m unabhängig voneinander für ganze Zahlen zwischen 5 und 40 stehen, mit der Maßgabe, daß n + m ≥ 38 ist;
a und b unabhängig voneinander für ganze Zahlen zwischen 1 und 10 stehen;
R und R' unabhängig voneinander ausgewählt sind aus -H und -CH₃;
X- ein physiologisch verträgliches Anion ist.

2. Haarbehandlungsmittel nach Anspruch 1, **dadurch gekennzeichnet, daß** es 0,25 bis 25 Gew.-%, vorzugsweise 0,5 bis 20 Gew.-%, weiter bevorzugt 1 bis 15 Gew.-%, noch weiter bevorzugt 1,5 bis 10 Gew.-% und insbesondere 2 bis 5 Gew.-% mindestens einer Verbindung der allgemeinen Formel (I) enthält.

3. Haarbehandlungsmittel nach einem der Ansprüche 1 oder 2, **dadurch gekennzeichnet, daß** es 0,25 bis 25 Gew.-%, vorzugsweise 0,5 bis 20 Gew.-%, weiter bevorzugt 1 bis 15 Gew.-%, noch weiter bevorzugt 1,5 bis 10 Gew.-% und insbesondere 2 bis 5 Gew.-% mindestens einer Verbindung der allgemeinen Formel (la) 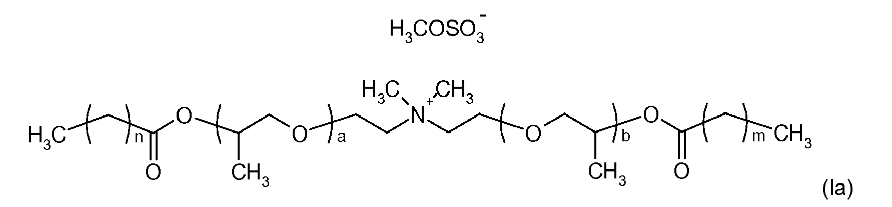 in der
n und m unabhängig voneinander für ganze Zahlen zwischen 5 und 40 stehen, mit der Maßgabe, daß n + m ≥ 38 ist;
a und b unabhängig voneinander für 1, 2, 3, 4 oder 5 stehen.

4. Haarbehandlungsmittel nach einem der Ansprüche 1 bis 3, **dadurch gekennzeichnet, daß** es 0,5 bis 20 Gew.-%, vorzugsweise 0,75 bis 15 Gew.-%, weiter bevorzugt 1 bis 10 Gew.-%, noch weiter bevorzugt 1,5 bis 7,5 Gew.-% und insbesondere 2 bis 4,5 Gew.-% mindestens einer Verbindung der allgemeinen Formel (Ib) 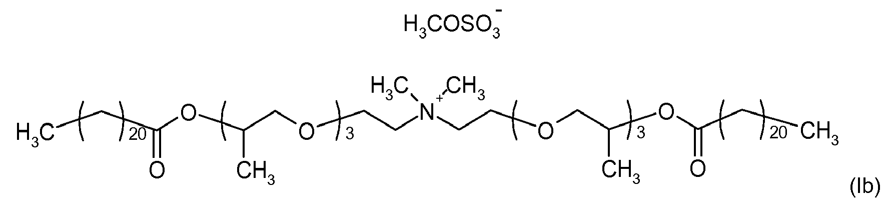 enthält.

5. Haarbehandlungsmittel nach einem der Ansprüche 1 bis 4, **dadurch gekennzeichnet, daß** es 2,5 bis 35 Gew.-%, vorzugsweise 3 bis 30 Gew.-%, weiter bevorzugt 5 bis 29 Gew.-%, noch weiter bevorzugt 7,5 bis 28 Gew.-% und insbesondere 12,5 bis 27 Gew.-% anionische(s) und/oder amphotere(s) und/oder nichtionische(s) Tensid(e) enthält.

6. Haarbehandlungsmittel nach einem der Ansprüche 1 bis 5, **dadurch gekennzeichnet, daß** es 2,5 bis 35 Gew.-%, vorzugsweise 5 bis 30 Gew.-%, weiter bevorzugt 7,5 bis 27,5 Gew.-%, noch weiter bevorzugt 10 bis 25 Gew.-% und insbesondere 12,5 bis 22,5 Gew.-% anionische(s) Tensid(e) enthält.

7. Haarbehandlungsmittel nach einem der Ansprüche 1 bis6, **dadurch gekennzeichnet, daß** es 1 bis 30 Gew.-%, vorzugsweise 1,5 bis 25 Gew.-%, weiter bevorzugt 2 bis 20 Gew.-%, noch weiter bevorzugt 2,5 bis 15 Gew.-% und insbesondere 3 bis 10 Gew.-% amphotere(s) Tensid(e) enthält.

8. Haarbehandlungsmittel nach einem der Ansprüche 1 bis 7, **dadurch gekennzeichnet, daß** es zusätzlich 0,1 bis 10 Gew.-% mindestens einer Verbindung der Formel (II) enthält 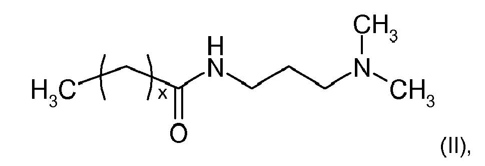 in der x für 18, 19, 20, 21, 22, 23 oder 24 steht.

9. Haarbehandlungsmittel nach einem der Ansprüche 1 bis 8, enthaltend
a. 0,5 bis 20 Gew.-%, vorzugsweise 0,75 bis 15 Gew.-%, weiter bevorzugt 1 bis 10 Gew.-%, noch weiter bevorzugt 1,5 bis 7,5 Gew.-% und insbesondere 2 bis 4,5 Gew.-% mindestens einer Verbindung der allgemeinen Formel (Ib) 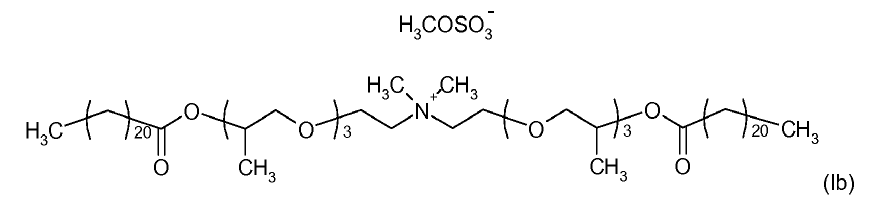
b. 0,1 bis 10 Gew.-% vorzugsweise 0,15 bis 5 Gew.-%, weiter bevorzugt 0,2 bis 2,5 Gew.-%, noch weiter bevorzugt 0,25 bis 1,5 Gew.-% und insbesondere 0,3 bis 1 Gew.-% mindestens einer Verbindung der Formel (Ila)

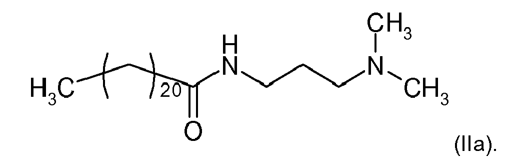

10. Verfahren zur Haarbehandlung, **dadurch gekennzeichnet, daß** ein Haarbehandlungsmittel nach einem der Ansprüche 1 bis 9 auf das Haar aufgebracht und nach einer Einwirkzeit von 5 Sekunden bis 15 Minuten wieder ausgespült wird.
